# EUROPEAN PATENT SPECIFICATION

(11) **EP 4 371 585 B1**
(45) Date of publication and mention of the grant of the patent: **11.03.2026**
(21) Application number: 24162643.1
(22) Date of filing: 25.04.2018
(51) Int. Cl.: A61B 90/00, A61L 27/16, A61L 27/18, A61L 27/36

(54) **MATERIALS AND METHODS FOR PROTECTING AGAINST NEUROMAS**
MATERIALIEN UND VERFAHREN ZUM SCHUTZ GEGEN NEUROME
MATÉRIAUX ET PROCÉDÉS DE PROTECTION CONTRE LES NÉVROMES

(30) Priority: 25.04.2017 US 201715496578
(43) Date of publication of application: 22.05.2024
(62) Divisional of application: 18791114.4
(73) Proprietor: Axogen Corporation, Alachua, FL 32615 (US)
(72) Inventor: DEISTER, Curt, Alachua, 32615 (US); SIMON, Crystal, Alachua, 32615 (US); FALERIS, Jennifer, Alachua, 32615 (US)
(74) Representative: Mathys & Squire

(56) References cited:
- WO-A1-2014/130419
- WO-A1-2016/144166
- US-A- 4 778 467
- US-A1- 2010 055 149
- US-A1- 2014 094 932

## Description

### BACKGROUND OF INVENTION

Neuromas develop as a part of a normal reparative process following peripheral nerve injury. They are formed when nerve recovery towards the distal nerve end or target organ fails and nerve fibers improperly and irregularly regenerate into the surrounding scar tissue. Neuromas consist of a deranged architecture of tangled axons, Schwann cells, endoneurial cells, and perineurial cells in a dense collagenous matrix with surrounding fibroblasts (Mackinnon SE et al. 1985. Alteration of neuroma formation by manipulation of its microenvironment. Plast Reconstr Surg. 76:345-53). The up-regulation of certain channels and receptors during neuroma development can also cause abnormal sensitivity and spontaneous activity of injured axons (Curtin C and Carroll I. 2009. Cutaneous neuroma physiology and its relationship to chronic pain. J. Hand Surg Am. 34:1334-6). Haphazardly arranged nerve fibers are known to produce abnormal activity that stimulates central neurons (Wall PD and Gutnick M. 1974. Ongoing activity in peripheral nerves; physiology and pharmacology of impulses originating from neuroma. Exp Neurol. 43:580-593). This ongoing abnormal activity can be enhanced by mechanical stimulation, for example, from the constantly rebuilding scar at the injury site (Nordin M et al. 1984. Ectopic sensory discharges and paresthesiae in patients with disorders of peripheral nerves, dorsal roots and dorsal columns. Pain. 20:231-245; Scadding JW. 1981. Development of ongoing activity, mechanosensitivity, and adrenaline sensitivity in severed peripheral nerve axons. Exp Neurol. 73:345-364).

Neuromas of the nerve stump are unavoidable consequences of nerve injury when the nerve is not, or cannot be, repaired and can result in debilitating pain. It has been estimated that approximately 30% of neuromas become painful and problematic. This is particularly likely if the neuroma is present at or near the skin surface as physical stimulation induces signaling in the nerve resulting in a sensation of pain.

Neuroma prevention and attenuation strategies have used various methods to limit neuroma size and protect the neuroma from external stimuli. Current prevention methods attempt to limit the size of the neuroma and so reduce or limit potential contact and "cross-talk" between axons within the injured nerve site and within the disorganized structure that characterizes neuromas. Due to a variety of factors, current methods of neuroma mitigation/prevention have an unacceptable level of efficacy.

While various methods have been used to prevent, minimize, or shield neuromas, the current clinical "gold standard" for treating neuromas is to bury the nerve end (that will form the neuroma) into muscle or a hole drilled in bone. The surrounding tissue cushions and isolates the neuroma to inhibit stimulation and the resulting painful sensations. However this procedure can greatly complicate surgery, as significant additional dissection of otherwise healthy tissue is required to emplace the nerve stump. For these reasons, emplacement of the nerve stump is often not performed in amputations (and many other nerve procedures).

Another method is to cut the nerve stump back to leave a segment or sleeve of overhanging epineurium. This overhang can be ligated to cover the face of the nerve stump. Alternatively, a segment of epineurium can be acquired from other nerve tissue or a corresponding nerve stump can be cut back to create an epineurium sleeve that can be used to connect with and cover the other nerve stump.

Yet another method that is commonly used is a suture ligation, where a loop of suture is placed around the end of the nerve and tightened. This pressure is believed to mechanically block the exit of axons and causes the terminal end to eventually form scar tissue over the site. Clinical and pre-clinical evidence has shown, however, that this procedure can cause a painful neuroma to form behind the ligation. Furthermore, the ligated nerve is generally not positioned to minimize mechanical stimulation of the neuroma, since it is anticipated that the scar tissue will provide sufficient protection to the nerve end.

Other methods include covering the nerve stump with a silicone or rubber tube; a vein, or a silicone rubber plug (*i.e.* a tube with a sealed end) have also been used. These devices and methods necessitate insertion of the nerve into the opening of the device, which can be difficult and can further damage the nerve end. These methods and devices also maintain the neuroma as a single mass, whereby stimulation in one area creates a cascade effect that can eventually encompass the entire neuroma mass. Thus, even minor stimulation of a neuroma can cause the entire neuroma mass to react.

Unfortunately, current methods for addressing the formation of and pain caused by neuromas have not been generally successful and therefore are rarely utilized. The formation of neuromas at a severed nerve end can be difficult to prevent. As such, methods and devices that can inhibit or alleviate the pain caused by neuromas can provide relief to patients.

US 2014/094932 A1 describes devices and methods for alleviating discomfort associated with neuroma formation. A cylindrical cap is provided wherein the internal chamber of the cylindrical cap physically partitions the nerve to enable an arrangement of nerve fibers (as opposed to haphazardly arranged nerve fibers often produced in neuromas).

### BRIEF SUMMARY

The subject invention provides devices and methods for manufacturing devices as defined by the claims. The devices can be for alleviating discomfort associated with neuroma formation. Specifically, the devices can be biomedical devices for neuroma size limitation and neuroma protection and isolation.

In preferred embodiments, the subject device is a cylindrical cap with a single open end to be applied to nerves within a subject's body, wherein the internal chamber of the cylindrical cap has dividers that can physically partition the nerve structures into distinct, and in some embodiments, separate channels to enable a more regular arrangement, rather than the haphazard arrangement often produced in neuromas. Preferably the device is sterile. The device has a single open end, such that the nerve end can be inserted into the internal chamber through the open end. There is a hollow indentation or retaining cavity between the open end and the dividers in which the terminal end of the nerve can be situated and secured. As the nerve grows, the axons and other structures will extend and grow into the separate channels to limit contact between them.

There is at least one panel divider within and attached to an internal surface of the internal chamber, wherein the at least one panel subdivides at least a portion of the internal chamber. The internal chamber is partitioned with at least one panel, directed longitudinally with the internal chamber, such that a portion of the internal chamber, opposite to the single open end, is divided into two or more distinct and separate channels. The division of the neuroma tissue into separate or subdivided channels, to form smaller, disconnected, neuroma masses, can mitigate the cascade effect caused when one area of the neuroma is stimulated and the effect is felt through the entire tissue mass. The dimensions of the cylindrical cap can be about 1 mm to about 25 mm in diameter. The external length of the cap is of mm to 500 mm.

In addition, the material of the cylindrical cap can remodel into a tissue cushion after implantation, thereby enveloping and further isolating the new neuronal growth, which protects the neuroma from being stimulated and inhibits pain. Remodeling can include, but is not limited to, expansion or enlargement of the material, reshaping of the material, incorporation of the material around nerve tissue, and other changes that can affect the size and shape of the material. Preferably, after the device is implanted, it will remodel into the surrounding tissue to protect the forming neuroma and the sections of smaller, disconnected neuromas mass. In one embodiment, the material of the cap is a biomaterial that can remodel to generate a volume of protective connective tissue around a neuroma (rather than the current use of a biomaterial to contain a neuroma volumetrically). In a preferred embodiment, the material of the cap is a membrane biomaterial, such as, for example, small intestine submucosa (SIS), amnion, dermis, or decellularized fascia.

The device contains an internal chamber with a retaining cavity at or near the open end. The internal chamber with the retaining cavity can allow insertion of the nerve stump through the open end, so that it is near or abuts against the divided, or separate, channels facing the open end. A dense layer of biomaterial on the exterior surface can operate to mechanically isolate the neuroma and prevent axons from escaping the internal chamber of the device. The internal chamber contains partitions or dividers of biomaterial to subdivide the neuroma mass **13** into smaller, disconnected neuroma masses **17** as it grows that will form from the nerve stump.

Insertion of a terminal nerve end into the internal channel can be difficult. A terminal nerve end is one that has been cut or transected leaving exposed axons and other internal nerve structures. Usually, either the outside of the cap and/or the epineurium around the nerve are held with a forceps so the nerve can be moved through the open end. Excessive pressure applied to the nerve can damage internal axons and structures. Likewise, excess force applied around the cap can distort the shape or the open end, inhibiting insertion of the nerve end. An open end in the cap that is too large can allow the neuroma to escape and grow outside the cap. If the open end is made smaller, the nerve can be damaged if forced into the open end.

To facilitate insertion of a terminal nerve end, the cap has one or more tabs arranged on and around the external surface **16** of the cap. In an alternative embodiment, a tab can be arranged at or near the open end. The tabs can be used to grasp the cap and manipulate it around the terminal nerve end. Grasping the tabs rather than the exterior surface can help to maintain the shape of the cap, in particular the open end.

The open end leading into a retaining cavity in which the terminal nerve end can be secured can also be made temporarily larger or more accessible, so that the terminal nerve end can be more easily and gently placed therein. In one embodiment, there is a slit in the cap that is integral with and perpendicular to the opening, such that the opening can be temporarily widened. In another embodiment, the retaining cavity has one or more wings or flaps that allow it to be partially spread open. This can widen the open end sufficiently that a terminal nerve end can be placed or set down within the retaining cavity, making it easier to see how it is placed relative to the dividers. The wings or flaps can then be wrapped over and around the nerve end and secured to hold the nerve in place and inhibit escape of neuronal tissue growth.

The combination of limiting the growth and size of a neuroma mass through physical partitioning of the neuromas mass into smaller, disconnected sections along with the creation of a connective tissue cushion is unique. This novel combination of features and design effectively uses the natural response of reconstructing implanted biomaterials to minimize the size, isolate, and protect the neuroma.

### BRIEF DESCRIPTION OF THE DRAWINGS

**Figures 1A, 1B and 1C** show a schematic presentation of consecutive steps of various current nerve stump capping methods: Step 1 - nerve transection; Step 2 - nerve stump preparation (A and B, nerve sheath must be slid off, then a piece of the nerve is removed to prepare a sleeve-like fragment of epineurium; C, cap can be formed of any autologous tissue, sutured to the epineurium); Step 3 - the end of epineurium can be tied up or sutured. Lewin-Kowalik J. et al. (2006) Prevention and Management of Painful Neuroma. Neural Med Chir (Tokyo), 46:62-68.
**Figures 2A, 2B and 2C** show perspective views (**2A and 2C**) of two embodiments of the invention and a side view (**2B**) of an embodiment of the device of the subject invention.
**Figures 3A and 3B** show a side cut-away view (Figure **3A**) of and top view of the proximal end (Figure **3B**) of the embodiment.
**Figure 4A** is a schematic drawing showing an untreated neuroma.
**Figure 4B** is a schematic drawing showing a neuroma treated using an embodiment of a device of the subject invention.
**Figure 5A** is a schematic drawing showing a perspective view of another embodiment of the device of the invention.
**Figure 5B** is a view illustrating a press-forming process executed by the press-forming apparatus to manufacture an embodiment of the device shown in Figure **5A****.**

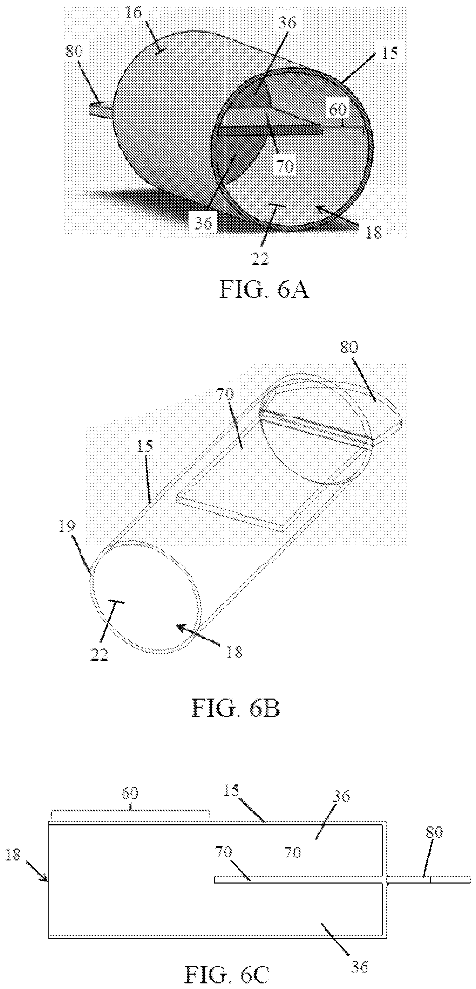

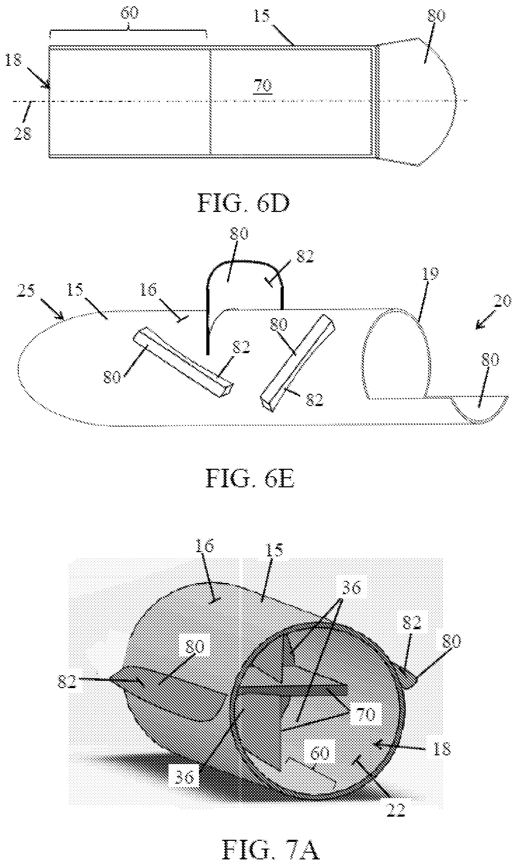

**Figures 6A, 6B, 6C****,** **6D****, and** **6E** illustrate embodiments of a cap, according to the subject invention, configured with a tab by which the cap can be manipulated and an internal panel that subdivides a portion of the distal end of the internal chamber into separate channels. Figure 6A is a proximal end perspective view of an embodiment. Figure 6B is top side, proximal end perspective view of the embodiment. Figure 6C is a right side elevation view of the embodiment. Figure 6D is a top side plan view of the embodiment. Figure 6E is a left side perspective view of an alternative embodiment of a cap with a tab extending from the edge of the open end.

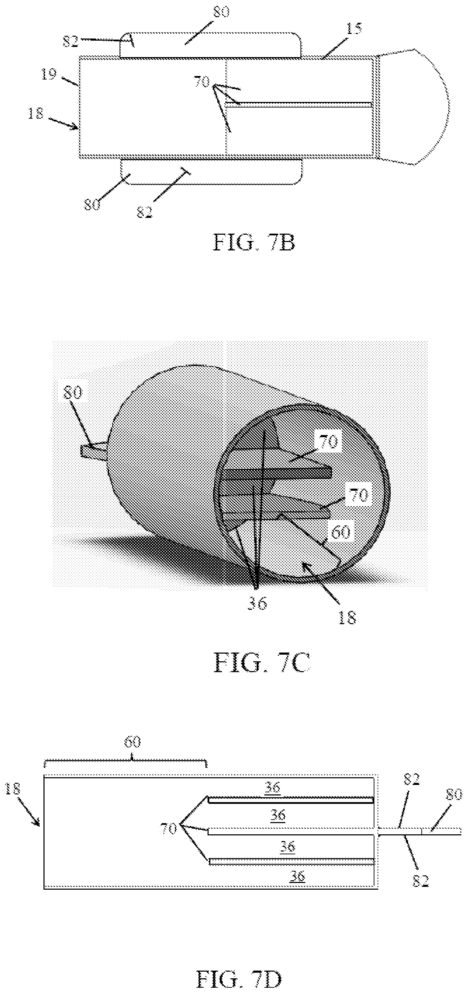

**Figures 7A****,** **7B****,** **7C** **and** **7D** illustrate an embodiment of a cap, according to the subject invention, configured with multiple tabs by which the cap can be manipulated and also shows several internal panels that subdivide a portion of the distal end of the internal chamber into multiple separate channels. The example shown in Figures 7A and 7B has a tab at the distal end and two tabs on the right and left sides. Also shown in this example are three perpendicularly placed panels within the internal chamber. Figures 7C and 7D illustrate an alternative embodiment where more than one panel is arranged parallel with each other in the internal cavity.

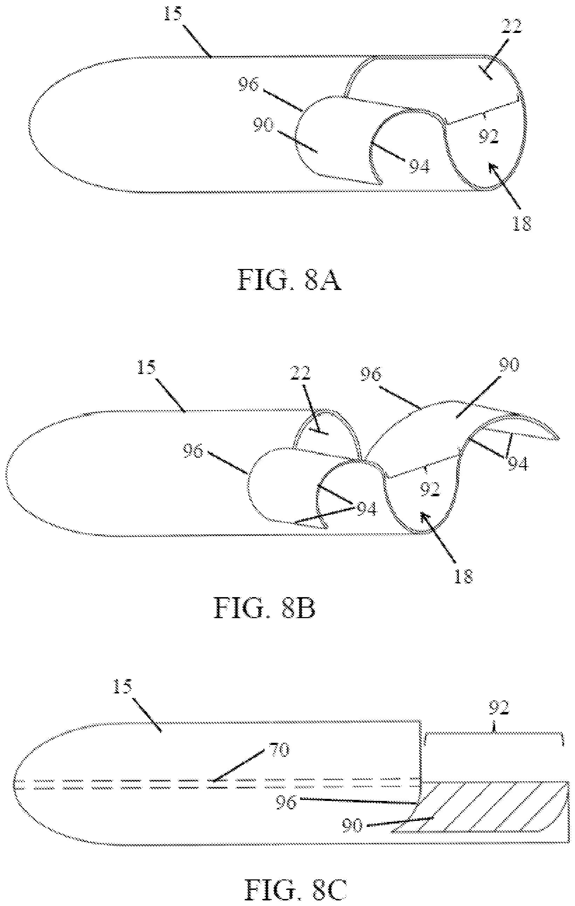

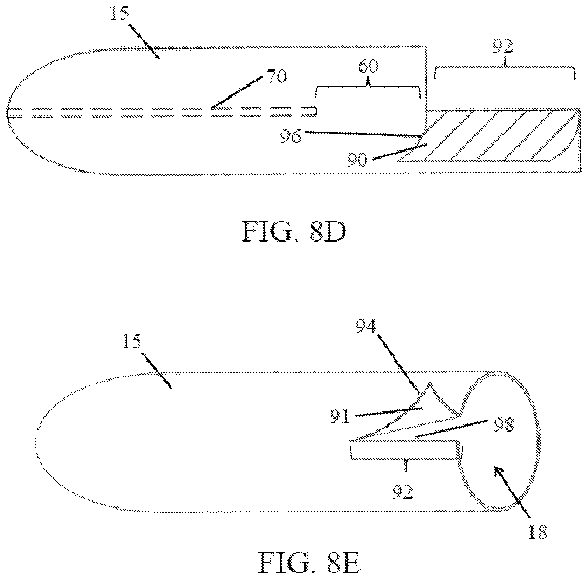

**Figures 8A, 8B, 8C****,** **8D,** and **8E** illustrate embodiments of a cap, according to the subject invention, where the open end is configured with one or more flaps that partially spread or widen the open end to allow a terminal nerve end to be more easily placed within the retaining cavity. Figure 8A shows an embodiment with one flap and Figure 8B shows an embodiment with two flaps. Figure 8C illustrates how distal edge of the flaps can open directly onto the separate channels. Figure 8D illustrates an alternative embodiment where the distal edges of the flaps open proximal to the separate channels. Figure 8E shows a non-limiting example of a cap with a slit at the open end, such that there is are tri-corner flaps that can be used to temporarily widen the open end.

### DETAILED DISCLOSURE

The subject invention provides devices and methods for manufacturing devices for alleviating discomfort associated with neuromas. More specifically, the subject invention pertains to devices and methods for limiting neuroma size and physically surrounding the neuroma to inhibit stimulation that elicits neuropathic pain. In certain embodiments, the subject invention is directed to a tissue-engineered scaffold that provides: a barrier that limits the size of a neuroma, dividers that subdivide the neuroma volume or mass into smaller, disconnected neuromas masses to reduce axonal cross-talk or the "cascade effect," and mechanical isolation of the neuroma to inhibit stimulation.

A device of the subject invention is designed to form a protective, connective tissue covering or cap surrounding the terminal nerve end **29,** thereby surrounding any resulting neuroma formation. Within the volume created by the barrier, sub-dividing the neuroma volume as it forms can limit the amount of interaction between axons, thereby limiting signaling cascades being triggered inside the neuroma that can be interpreted as pain. By providing mechanical isolation of the neuroma volume, the subject device limits stimulation of the neuroma. The ability of the material of the subject device to remodel into a native tissue cushion is another advantageous feature when combined with the physical partitioning of the neuroma.

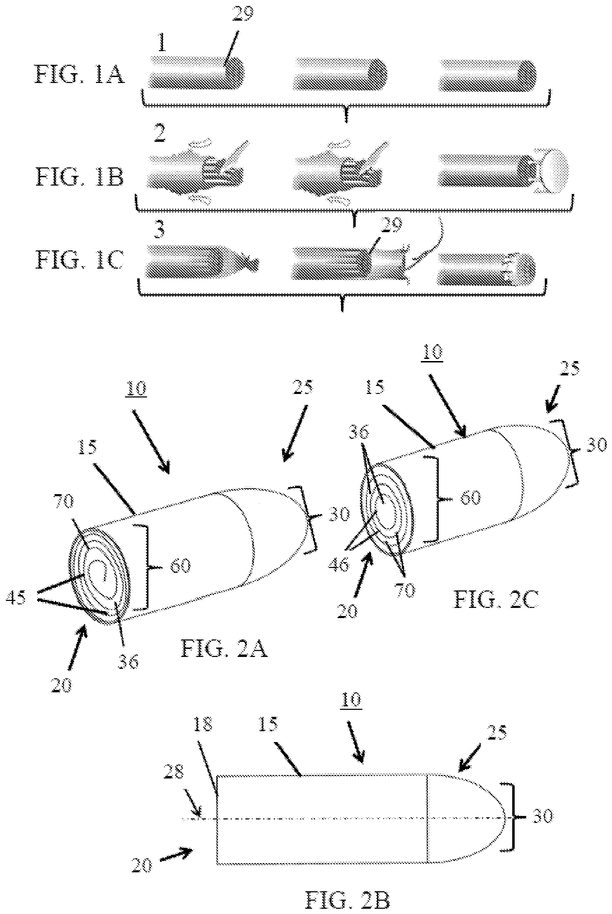

Current methods for limiting the size and stimulation of a neuroma are shown in Figures 1A-1C. The methods include dissecting or cutting the terminal end to form a flap of epineurium that can be folded over the terminal nerve end **29,** as shown in the first column of Figures 1A-1C. The nerve can also be dissected or cut back to form a sleeve of epineurium that can be ligated over the terminal nerve end, as seen in the middle column in Figures 1A-1C. A patch epineurium can be obtained from another location and attached to the terminal nerve end, as shown in the last column in Figures 1A-1C.

The subject invention provides the benefits of an epineural flap (or ligation) to limit neuroma size and the advantages of isolating a nerve end by emplacement in another tissue combined in a single device. This device isolates and protects the neuroma, thereby inhibiting painful sensations without the need for any repositioning of the nerve. Advantageously, the device can be secured to the stump of the nerve prior to formation of a neuroma and as the neuroma forms the material of the device can remodel into a tissue cushion **55** and become integrated into the surrounding host tissues, which effectively isolates the neuroma from undesirable contact and stimulation.

Figures 2A-8B illustrate embodiments of the invention.. In one embodiment, illustrated in Figure 2, the cap **10** of the subject invention has an external body **15** that comprises a proximal end **20** and a distal end **25.**

It can be seen in Figure 2 that one embodiment of the external body **15** of the cap **10** is cylindrical in shape. However, the shape of the external body **15** of the cap **10** can vary depending upon the type, diameter and location of a nerve stump, as well as other factors known to those with skill in the art. For example, the circumferential shape of the external body can be variable and be, by way of non-limiting examples, an ovoid, a circle, a square, a rectangle, a triangle, or any other polygonal shape. In an exemplified embodiment, the circumferential shape of the external body is generally a circle.

The dimensions of the external body can vary depending on the type, diameter and location of a nerve stump on which it will be used, as well as other factors known to those skilled in the art. In one embodiment, the external body of the device has dimensions of approximately 1mm to approximately 100 mm in diameter and is approximately 1 mm to approximately 500 mm in length, between the proximal end **20** and the distal end **25.** In a particular embodiment, the external body of the device will be less than 100 mm in diameter and less than 500 mm in length. In a specific embodiment, the diameter of the external body is between approximately 1 mm and approximately 25 mm and the length of the external body, between the proximal end and the distal end of the cap, is between approximately 1 mm and approximately 100 mm.

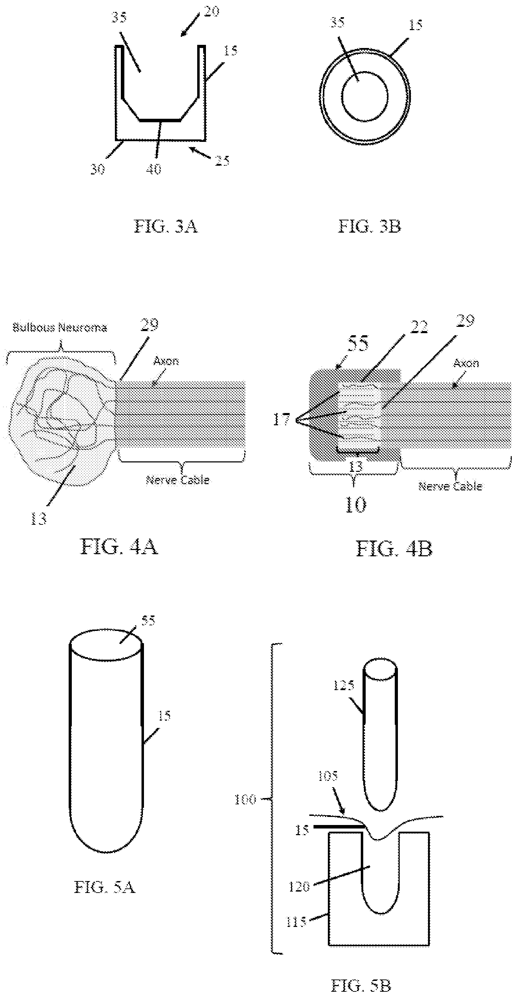

In one embodiment, the diameter of the distal end **25** of the external body **15** can be narrower than the diameter of the proximal end **20,** as seen, for example, in Figures 2 and 3. This can give the cap a bullet or conical shape. In a particular embodiment, shown for example in Figures 2A-2C, the distal end **25** of the external body is cone-shaped. In an alternative embodiment, shown for example in Figures 6A-6E and Figures 7A-7B, the diameter of the distal end and the proximal end are the same or substantially the same, such that the distal end ofthe cap is flat, giving the cap a cylinder or barrel shape.

Ideally, the external body **15** of the cap **10** has no openings other than the open end **18** at the proximal end **20** of the cap through which a nerve can be inserted or placed. In one embodiment, for example, as shown in Figure 3, the proximal end **20** is open and the distal end **25** of the external body **15** is closed off entirely so that the external body forms a cap **10.** Figure 3 also illustrates an embodiment in which an internal chamber **35** is provided in the external body **15.** In certain embodiments, the interior surface **22**of the closed off **30** portion of external body **15** can be in the form of a bevel **40,** as shown in Figure 3A. Alternatively, a closed off portion can be substantially flat, as shown in Figures 6C and 7A. In yet another alternative, the closed off portion or closed end **30** can be conical, such as shown, for example, in Figures 2A and 6E .

An external body without excess openings can inhibit axon growth from extending outside the cap. The open end **18** of the cap can provide the only access into the internal chamber. Fitting a terminal nerve end **29** through the open end of a cap can be challenging and can potentially damage the nerve. If a cap with a larger diameter is selected, it can be easier to insert the terminal nerve end, but may result is excess space around the nerve perimeter. If not properly closed off, the openings around the nerve can allow axons to escape.

The use of flaps **90** around the open end **18** can provide a temporary enlargement of the open end, making nerve placement into the internal chamber easier, while the flap can be wrapped around and sutured to provide for a close fit around the terminal nerve end. The enlargement of the open end allows a terminal nerve end to be moved through a slot **92** formed in the open end. Figures 8A and 8E illustrate non-limiting examples of a cap having an open end **18** with one or more flaps. Ideally, the slot provided by one or more flaps is large enough to allow a terminal nerve end to pass through the slot, with minimal compression of the nerve tissue, so as to minimize the number of sutures necessary to close the flap and the slot.

In one embodiment, there is a single flap, as shown in Figure 8A. The single flap can provide a slot of between approximately 25% to approximately 75% of the diameter of an opening **18.** In a more particular embodiment, there is a single flap that provides a slot of between approximately 35% to approximately 65% of the diameter of the opening. In a specific embodiment, there is a single flap that provides a slot of between approximately 45% to approximately 55% of the diameter of the opening. In a specific embodiment, there is a single flap that provides a slot of approximately 50% of the diameter of the opening.

In an alternative embodiment, there are two flaps, as shown in Figure 8B. The dual flaps can open in opposite directions. The dual flaps can provide a slot of between approximately 25% to approximately 75% of the diameter of an opening **18.** In a more particular embodiment, there is a single flap that provides a slot of between approximately 35% to approximately 65% of the diameter of the opening. In a specific embodiment, there is a single flap that provides a slot of between approximately 45% to approximately 55% of the diameter of the opening. In a specific embodiment, there is a single flap that provides a slot of approximately 50% of the diameter of the opening.

In an alternative embodiment, there is a slit **98** in the proximal end **20** of the cap that is integral with opening **18** and that advances towards the distal end **25** to form a flap with three corners part-way along the external body **15** of the cap. Figure 8E illustrates a non-limiting example of a three-corner flap.

The nerve tissue in a neuroma mass **13** grows in a random, haphazard fashion and the axons in one section of a neuroma can be in contact with other surrounding axons. This creates the disadvantageous effect of the neuroma acting as one, open nerve end. As a result, stimulation of a neuroma in one area can trigger a cascade effect wherein the signal received by axons in one area, because of contact, will trigger signals in nearby axons. Each step in the cascade can amplify the initial signal until finally most or all of the axons in the neuroma are "fired." This massive signal cascade can also amplify pain. By isolating or separating areas of the neuroma from other areas of the neuroma, this cascade effect can be minimized, thereby limiting the amount of pain caused by stimulation of the neuroma.

The internal chamber **35** is configured with one or more dividers 70 that subdivide at least a portion of the internal chamber into two or more separate channels 36 into which the neuroma mass **13** can grow and become portioned into smaller, disconnected neuroma masses. This separation of the neuroma mass into the different channels creates a physical barrier between the axons of the smaller, disconnected portions in the different separate channels, thereby limiting the scope of a cascade effect. The dividers **70** used in an internal chamber can be configured in a myriad of ways to create different sizes and shapes of separate chambers. Likewise, the dividers can be individual or unconnected or they can be joined together. Variations in the configurations of the dividers that separate a neuroma mass into smaller, disconnected masses **17** or portions with different channels are within the scope of this invention.

A nerve end can be positioned within the cap **10** so that the neuronal growth is directed towards the closed end **30** and the separate chambers. It can be beneficial if there is some overlap between the external body and the epineurium, so as to provide space or distance between the end of the nerve and the open end when the terminal nerve end **29** is secured in the cap. This can be achieved by having the dividers **70** terminate some distance from the open end, thereby forming a retaining cavity **60** between the open end **18** and a divider. Figures 6A, 6C, 7A, and 7D illustrate non-limiting examples of dividers that are shorter than the length, between the distal end and the proximal end of the cap, of the external body **15.** The termination of the dividers before the open end forms an undivided retaining cavity in which the terminal nerve end can be disposed. The subsequently forming neuroma will be directed towards the separate channels and divided into separate, smaller, disconnected portions or volumes as it grows into the separate channels.

A divider is shorter than length of the internal chamber, such that between approximately 25% to approximately 75% of the internal chamber is divided into separate channels. In a more particular embodiment, a divider is shorter than the length of the internal chamber, such that between approximately 40% and 60% of the internal chamber is divided into separate channels. In a specific embodiment, a divider is shorter than the length of the internal chamber, such that approximately 50% of the internal chamber is subdivided into separate chambers.

According to one embodiment , which is not according to the invention as claimed, invention, the external body **15** can have an internal chamber **35** that includes spiral partitions **45.** Advantageously, the spiral partitions enable subdivision and arrangement of axons from the nerve stump. In certain related embodiments, the internal chamber of the external body comprises a tightly packed spiral of a solid sheet of biomaterial. Preferably, where the internal chamber of the external body comprises a tightly packed spiral of a solid sheet of biomaterial, there are no voids present that could lead to axonal escape from the device.

In an alternative embodiment, which is also not according to the invention as claimed, the internal chamber can be subdivided by separate concentric tubes **46,** such as shown, for example, in Figure 2C. The concentric tubes **46** can also partition or subdivide the internal chamber, enabling the formation of smaller, disconnected neuromas masses **17.** In another embodiment, the divider **70** is a panel that extends across and attaches to the internal surface **22** of the internal chamber **35.** The panel divider can extend from the closed portion **30** at distal end **25** and towards the open end **18** proximal end **20** thereby dividing the closed off portion **30** of the internal chamber in a longitudinal direction **28** into two separate channels **36.** In a further embodiment, the panel divider extends partially through the internal chamber, such that a portion of the internal chamber is subdivided and another portion, nearer the proximal end, remains undivided. Figures 6A-6D illustrate a non-limiting example of this embodiment. In a further embodiment, there can be more than one panels so that the internal chamber is divided into more than two separate channels. Figures 7A and 7B illustrate non-limiting examples of 2 and 3 panels arranged perpendicularly, dividing the internal chamber into four quadrants. Figures 7C and 7D illustrate another non-limiting example of multiple panels arranged parallel within the internal chamber, dividing the internal chamber into similar parallel, separate channels. Other arrangements of panels that form different configurations of separate channels are within the scope of this invention.

A panel extends in a longitudinal direction **28** between approximately ¼ to approximately ¾ of the length, between the proximal end **20** and the distal end **25** of the internal chamber. In a more specific embodiment, a panel extends in a longitudinal direction between approximately 2/5 to approximately 3/5 of the length of the internal chamber. In a specific embodiment, a panel extends approximately ½ of the length of the internal chamber.

The flaps **90,** mentioned above, can provide a slot **92** that allows the terminal nerve end **29** to be placed in close proximity to the dividers **70.** In one embodiment, the flaps have a rear edge **96** that allows the flap to open onto the dividers, so that the slot is directly open to the dividers, allowing a terminal nerve end to be placed against the dividers, which is shown, for example, in Figure 8C. In another embodiment, the flaps have a rear edge **96** that is proximal to the dividers, such that the flap opens more proximal **20** to the dividers, providing for at least a small retaining cavity **60** between the rear edge or the slot and the dividers, such as shown, for example, in Figure 8D.

In an alternative embodiment, the flap is formed by a slit **98** in the external body of the cap, extending from the opening towards the distal end. Figure 8E illustrates a non-limiting example of a flap formed by a single slit **98** in the external body. The slit can extend part-way towards the distal end, such that it terminates at the dividers **70,** such that the slot **92** opens onto the dividers. Alternatively, the slit **98** can terminate proximal to the dividers, such that the slot **92** opens more proximal or a distance in front of to the dividers, providing for at least small retaining cavity **60** between the dividers and the end of the distal end of the slit.

In one embodiment, as illustrated in Figure 4B, the internal chamber **35** is an unchambered or undivided layer of biomaterial **50.** The biomaterial preferably isolates the neuroma and prevents axons from escaping the external body **15** of the device. Figure 4A is a schematic drawing showing an untreated neuroma.

Both natural and synthetic biomaterials can be used to manufacture a cap and components thereof of the subject invention. In certain embodiments, the biomaterial is a homogenous material. Examples of biomaterials for use in manufacturing the subject invention include, but are not limited to, high density polyethylene (HDPE), polyethylene glycol (PEG) hydrogel, purified proteins from human or animal sources (*e.g*., membrane of purified collagen or fibrin), and decellularized tissue constructs (*e.g*., demineralized bone, amnion, SIS, dermis, or fascia). An HDPE or PEG device can comprise or consist of a cylinder of porous HDPE or PEG surrounded by a layer of non-porous HDPE or PEG. Biomaterials that can form a fluid material, such as soluble purified collagen or particulate SIS and dermis, can be directly cast to form the device without a membrane as an intermediate.

In certain embodiments, which are not according to the invention as claimed, the external body **15** of the device can be made by rolling a sheet of biomaterial to form spiral partitions. Where the external body of the device is a "roll" of spiral partitions, the layers of the roll separate slightly to allow nerve regeneration to proceed a short distance into the device before encountering, and being stopped by, infiltrating non-nerve tissue (*i.e.,* the rolled version has longitudinal pores or characteristics). In specific related embodiments, layers of the rolled biomaterial are situated such that a spiral channel is present on the face of the device facing the nerve stump, while the device face external to the nerve stump is solid.

In other embodiments, the external body of the device can be made of a porous biomaterial. In yet other embodiments, the body includes a hollow central cavity to facilitate insertion of a nerve stump. In certain other embodiments, a body is provided with a hollow cavity wherein layers of biomaterial scaffolding fill a portion of the hollow cavity to create a laminar or multi-laminar construct **60** (see Figure 2C).

In an alternate embodiment, as illustrated in Figure 5A, the external body **15** of the device resembles a test tube, where the body is made of a thin layer of biomaterial (either a single layer or multiple layers) and there is a hollow central cavity **55** to facilitate insertion of a nerve stump.

Illustrated in Figure 5B is a method for producing the embodiment of the device shown in Figure 5A. Figure 5B illustrates a press formation apparatus **100** for manufacturing the device of Figure 5A. As shown in Figure 5A, a thin layer of biomaterial **105** (formed from either a single layer or multiple layers of biomaterial) is mounted on a receiving portion **115** of the press formation apparatus **100.** The receiving portion **115** has an opening **120** for receiving a punch **125,** where the shape of the opening **120** corresponds with that of the punch **125.** The receiving portion **115** and the punch **125** can be manufactured by die molding. Preferably, the punch **125** is rod shaped; however, other shapes known to the skilled artisan can be used for the opening of the receiving portion and the punch.

The thin layer of biomaterial **105** is arranged over the opening **120** of the receiving portion **115.** Once the biomaterial **105** is positioned, the punch **125** is then driven downward and received in the opening **120.** In this way, the body **15** of the device is formed.

In further embodiments, a cylindrical body having a hollow interior with open ends is provided. The distal end of the body may be "crimped" during the manufacturing process to present a more solid biomaterial as a barrier to axonal escape from the device. An example is the use of a crimped mold during vacuum pressing of a rolled cylinder, such that one end is of a smaller diameter.

In a preferred embodiment, the body of the subject device comprises a cylinder of SIS. A hollow cavity is provided at the proximal end to allow insertion of a nerve stump. A deep spiral partition is present in the body to subdivide the neuroma that will form from the nerve stump, and a dense layer of biomaterial is provided on the entire exterior surface to mechanically isolate the neuroma and prevents axons from escaping the device.

In a method of use, a nerve stump is secured in the internal chamber **35**by means of a suture, staple, clip, or surgical adhesive or sealant. After implantation, the cap is remodeled into the body's own tissue and provides a tissue cushion **55** for the neuroma. As host cells infiltrate the biomaterial, it is converted into a form of connective tissue. Axons and Schwann cells will also infiltrate from the stump. As fibroblasts (and other cells supporting remodeling into a connective tissue) 1) migrate and proliferate faster than Schwann cells/axons and 2) infiltrate from multiple sides and 3) axonal regeneration stops when it encounters other tissues (such as muscle, connective tissue layers, etc); the device will result in a layer of connective tissue surrounding a small neuroma in a vascularized tissue capsule. This capsule provides the desired isolation and protection.

It can be beneficial for the nerve end to be fully encapsulated within the cap, so that when the nerve begins to grow and form a neuroma, all neuronal growth is contained within the cap and, preferably, directed towards the separate channels at the closed end **30.** Manipulation of a terminal nerve end **29** into a cap can be difficult. Nerve tissue is usually soft and susceptible to damage if too much force is applied. Typically, the cap is manipulated onto the terminal nerve end. The cap can be grasped or encircled by various instruments to manipulate it onto the terminal nerve end. This can distort the shape of the external body and make it difficult to manipulate onto the terminal nerve end.

The external body **15** is configured with one or more tabs **80** extending out from the external body. The tabs can provide points for grasping the cap without contacting, or by making minimal contact, with the external body. A tab can be any size or shape and there can be more than one tab on an external body. Preferably, the size and shape of a tab provides opposing surfaces **82** for grasping with a forceps or any other instrument with pincers that can be squeezed together to grasp or hold small structures. A tab can also be used as a connection point, wherein by the cap and a terminal nerve secured therein can be attached to another structure in the body using a tab. In one embodiment, a tab comprises the same or similar material as the cap. For example, a tab can be formed from the same biomaterial that the cap is formed from, allowing the tab to be remodeled with the cap, as described above. In an alternative embodiment, the tab comprises a different material than the cap. For example, a tab can comprise a polymer that dissolves or is absorbed by the body. After the tab has been used to manipulate the cap into position, the tab can be left in place on the cap. Alternatively, a tab can be removed from the cap, such as by cutting with a scissors, blade, or compression sealing.

In one embodiment, a tab extends out from the closed end **30** of the cap **10,** such as shown, for example, in Figures 6B, 6C, and 6D. In these Figures, the tab is shown traversing the full diameter of the closed end. This is not required and the width of the tab can be less than the diameter of the cap, which is not shown, but is understood by a person with skill in the art.

In another embodiment, a tab can extend from the open end **18** of the cap **10,** such that it forms a lip or shelf around the edge **19** of the open end. Figure 6E illustrates one example of a tab **80** extending from the edge **19** of the open end **18.** This type of tab can be used to pull the cap onto a terminal nerve end. A tab extending from the edge of the open end can also support a terminal nerve end placed thereon and other tabs on the external body **15** can be used to push the cap onto the terminal nerve end.

In addition to tabs that can be used to push or pull a cap onto a terminal nerve end **29,** tabs can be located in and extend from other areas of the cap and used to twist, turn, rotate, or otherwise manipulate a cap **10** onto a terminal nerve end. In another embodiment, at least one tab extends out from or away from a side of the external body. One example of this is shown in Figures 7A and 7B, where two tabs are shown extending out from each side of the external body.

Furthermore, tabs can be arranged in any of a myriad of orientations on the external body. One embodiment, shown in Figure 7B, has at least one tab arranged with an opposing surface parallel with, or approximately parallel with, the longitudinal direction **28** of the internal chamber **35.** Figure 6E shows an alternative embodiment where the tab is arranged with an opposing surface perpendicular, or approximately perpendicular, to the longitudinal direction of the internal chamber. Tabs can also be arranged with an opposing surface, relative to the opening **18,** at an oblique angle with the longitudinal direction of the internal chamber. A person with skill in the art will be able to determine, for one or more tabs, the appropriate location on the external body and orientation with respect to the longitudinal direction of the internal chamber. Such variations are within the scope of this invention.

Neuroma formation occurs in approximately 30% of severed nerve ends. Without intervention, neuroma formation can form a randomly arranged conglomeration of axons. This haphazard arrangement of axons makes the neuroma susceptible to a cascade effect when the neuroma is stimulated, where stimulation in one area radiates and intensifies through the entire neuroma mass, resulting in intense pain. The embodiments of the subject invention can mitigate the effects of a cascade effect with caps that can be attached to terminal nerve ends. The caps can aid in reducing the size of a neuroma and can provide channels for mechanical separation of a neuroma mass, thereby limiting the effect of stimulation of the neuroma. The caps of the subject invention also have external tabs and flaps that can aid in fitting the cap over a terminal nerve end. The caps of the subject invention represent a significant improvement in amelioration of the pain caused by neuromas.

## Claims

1. A nerve cap comprising:
a body having a distal end, a proximal end with a single opening, and an internal chamber between the distal end and the proximal end, where the body comprises a biomaterial configured to remodel around a terminal nerve end to form a tissue cushion;
at least one panel divider within and attached to an internal surface of the internal chamber, wherein the at least one panel subdivides at least a portion of the internal chamber, wherein the at least one panel divider comprises a biomaterial, and wherein the at least one panel divider extends in a longitudinal direction from the distal end of the internal chamber approximately 1/4 to approximately 3/4 of a length of the internal chamber;
a retaining cavity between the single opening at the proximal end and the at least one panel divider; and
at least one tab extending from an external side of the body,
wherein an external length of the cap is in a range of 1 mm to 500 mm.

2. The nerve cap according to claim 1, wherein the at least one panel divider extends in the longitudinal direction approximately 2/5 to approximately 3/5 of the length of the internal chamber, or approximately 1/2 of the length of the internal chamber.

3. The nerve cap according to claim 1 or claim 2, wherein the at least one panel divider is single divider.

4. The nerve cap according to claim 3, wherein the single divider bisects the internal chamber.

5. The nerve cap according to claim 1 or claim 2, wherein the at least one panel divider includes two panels arranged perpendicular to each other.

6. The nerve cap according to claim 1 or claim 2, wherein the at least one panel divider includes two or more panels arranged parallel to each other.

7. The nerve cap according to any one of claims 1, 2, or 6, wherein the at least one panel divider includes two or more panels unconnected from each other.

8. The nerve cap according to any one of the preceding claims, wherein the at least one tab traverses a diameter of the distal end of the body.

9. The nerve cap according to any one of claims 1-7, wherein a width of the at least one tab is less than a diameter of the distal end of the body.

10. The nerve cap according to any one of the preceding claims, wherein the at least one tab includes a single tab.

11. The nerve cap according to any one of claims 1-9, wherein the at least one tab includes two or more tabs.

12. The nerve cap according to any preceding claim, wherein a material that forms the at least one tab is the same as a material that forms the cap.

13. The nerve cap according to any one of claims 1-11, wherein a material that forms the at least one tab is different from a material that forms the cap.

14. The nerve cap according any one of claims 1-11, wherein the at least one tab is made of a polymer that is configured to dissolve and/or be absorbed by the body.

15. The nerve cap according to any one of the preceding claims, wherein the at least one tab extends along an axis that is approximately parallel to or is parallel to the longitudinal direction of the internal chamber.

16. The nerve cap according to any one of claims 1-14, wherein the at least one tab extends along an axis that is approximately perpendicular to or is perpendicular to the longitudinal direction of the internal chamber.

17. The nerve cap according to any one of claims 1-14, wherein the at least one tab extends along an axis that is at an oblique angle relative to the longitudinal direction of the internal chamber.

18. The nerve cap according to claim 1, wherein the biomaterial is selected from the group consisting of: high density polyethylene (HDPE), polyethylene glycol (PEG) hydrogel, and purified proteins from human or animal sources.

19. The nerve cap according to claim 1, wherein the biomaterial is selected from the group consisting of: small intestine submucosa (SIS), amnion, dermis, collagen and decellularized fascia.

20. The nerve cap according to any one of claims 1-8 or 11-19, wherein the at least one tab extends from a side of the body.

21. The nerve cap according to any one of claims 1-19, wherein the at least one tab extends from the distal end of the body.

22. The nerve cap according to any one of claims 1-7 or 10-19, wherein the at least one tab is located at or near the single opening in the body, and optionally, the at least one tab extends from an edge of the single opening in the body.

23. The nerve cap according to any one of the preceding claims, wherein one or both of a length of the cap and a diameter of the cap is in a range of 1 mm to 100 mm.

24. A method of manufacturing a nerve cap, preferably according to any one of the preceding claims, comprising:
press forming a layer of biomaterial, preferably into a rod shape,
optionally wherein press forming the layer of biomaterial comprises:
mounting a layer of biomaterial on a receiving portion of a press formation apparatus, wherein the receiving portion has an opening for receiving a punch, wherein the shape of the opening corresponds with that of the punch;
arranging the layer of biomaterial over the opening of the receiving portion; and
once the biomaterial is positioned, driving the punch downward to be received in the opening.

## Patentansprüche

1. Eine Nervenkappe, die Folgendes umfasst:
einen Körper, der ein distales Ende, ein proximales Ende mit einer einzelnen Öffnung und eine innere Kammer zwischen dem distalen Ende und dem proximalen Ende aufweist, wobei der Körper ein Biomaterial umfasst, das dazu ausgelegt ist, sich um eine terminale Nervenendigung herum umzugestalten, um ein Gewebepolster zu bilden;
mindestens ein Plattenteilerelement innerhalb einer und befestigt an einer Innenfläche der inneren Kammer, wobei die mindestens eine Tafel mindestens einen Abschnitt der inneren Kammer unterteilt, wobei das mindestens eine Plattenteilerelement ein Biomaterial umfasst und wobei sich das mindestens eine Plattenteilerelement in einer Längsrichtung von dem distalen Ende der inneren Kammer ungefähr 1/4 bis ungefähr 3/4 einer Länge der inneren Kammer erstreckt;
einen Rückhaltehohlraum zwischen der einzelnen Öffnung an dem proximalen Ende und dem mindestens einen Plattenteilerelement; und
mindestens eine Lasche, die sich aus einer äußeren Seite des Körpers erstreckt,
wobei eine äußere Länge der Kappe in einem Bereich von 1 mm bis 500 mm liegt.

2. Nervenkappe nach Anspruch 1, wobei sich das mindestens eine Plattenteilerelement in der Längsrichtung ungefähr 2/5 bis ungefähr 3/5 der Länge der inneren Kammer oder ungefähr 1/2 der Länge der inneren Kammer erstreckt.

3. Nervenkappe nach Anspruch 1 oder Anspruch 2, wobei das mindestens eine Plattenteilerelement ein einzelnes Teilerelement ist.

4. Nervenkappe nach Anspruch 3, wobei das einzelne Teilerelement die innere Kammer zweiteilt.

5. Nervenkappe nach Anspruch 1 oder Anspruch 2, wobei das mindestens eine Plattenteilerelement zwei Tafeln einschließt, die senkrecht zueinander angeordnet sind.

6. Nervenkappe nach Anspruch 1 oder Anspruch 2, wobei das mindestens eine Plattenteilerelement zwei oder mehr Tafeln einschließt, die parallel zueinander angeordnet sind.

7. Nervenkappe nach einem der Ansprüche 1, 2 oder 6, wobei das mindestens eine Plattenteilerelement zwei oder mehr Tafeln einschließt, die nicht miteinander verbunden sind.

8. Nervenkappe nach einem der vorhergehenden Ansprüche, wobei die mindestens eine Lasche einen Durchmesser des distalen Endes des Körpers durchquert.

9. Nervenkappe nach einem der Ansprüche 1-7, wobei eine Breite der mindestens einen Lasche weniger als ein Durchmesser des distalen Endes des Körpers beträgt.

10. Nervenkappe nach einem der vorhergehenden Ansprüche, wobei die mindestens eine Lasche eine einzelne Lasche einschließt.

11. Nervenkappe nach einem der Ansprüche 1-9, wobei die mindestens eine Lasche zwei oder mehr Laschen einschließt.

12. Nervenkappe nach einem der vorhergehenden Ansprüche, wobei ein Material, das die mindestens eine Lasche bildet, das gleiche ist wie ein Material, das die Kappe bildet.

13. Nervenkappe nach einem der Ansprüche 1-11, wobei ein Material, das die mindestens eine Lasche bildet, verschieden von einem Material ist, das die Kappe bildet.

14. Nervenkappe nach einem der Ansprüche 1-11, wobei die mindestens eine Lasche aus einem Polymer hergestellt ist, das dazu ausgelegt ist, sich aufzulösen und/oder von dem Körper absorbiert zu werden.

15. Nervenkappe nach einem der vorhergehenden Ansprüche, wobei sich die mindestens eine Lasche entlang einer Achse erstreckt, die ungefähr parallel oder parallel zu der Längsrichtung der inneren Kammer ist.

16. Nervenkappe nach einem der Ansprüche 1-14, wobei sich die mindestens eine Lasche entlang einer Achse erstreckt, die ungefähr senkrecht oder senkrecht zu der Längsrichtung der inneren Kammer ist.

17. Nervenkappe nach einem der Ansprüche 1-14, wobei sich die mindestens eine Lasche entlang einer Achse erstreckt, die in einem relativ zu der Längsrichtung der inneren Kammer schrägen Winkel steht.

18. Nervenkappe nach Anspruch 1, wobei das Biomaterial aus der Gruppe ausgewählt ist, die aus Folgenden besteht: Polyethylen hoher Dichte (HDPE), Polyethylenglycol(PEG)-Hydrogel und gereinigten Proteinen aus menschlichen oder tierischen Quellen.

19. Nervenkappe nach Anspruch 1, wobei das Biomaterial aus der Gruppe ausgewählt ist, die aus Folgenden besteht: Dünndarmsubmukosa (SIS), Amnion, Dermis, Kollagen und dezellularisierten Faszien.

20. Nervenkappe nach einem der Ansprüche 1-8 oder 11-19, wobei sich die mindestens eine Lasche aus einer Seite des Körpers erstreckt.

21. Nervenkappe nach einem der Ansprüche 1-19, wobei sich die mindestens eine Lasche aus dem distalen Ende des Körpers erstreckt.

22. Nervenkappe nach einem der Ansprüche 1-7 oder 10-19, wobei sich die mindestens eine Lasche an der oder in der Nähe der einzelnen Öffnung in dem Körper befindet und wobei sich die mindestens eine Lasche optional aus einem Rand der einzelnen Öffnung in dem Körper erstreckt.

23. Nervenkappe nach einem der vorhergehenden Ansprüche, wobei eines oder beides von einer Länge der Kappe und einem Durchmesser der Kappe in einem Bereich von 1 mm bis 100 mm liegt.

24. Ein Verfahren zum Herstellen einer Nervenkappe, bevorzugt nach einem der vorhergehenden Ansprüche, das Folgendes umfasst:
Pressformen einer Biomaterialschicht bevorzugt zu einer Stabgestalt,
wobei das Pressformen der Biomaterialschicht optional Folgendes umfasst:
Anbringen einer Biomaterialschicht auf einem aufnehmenden Abschnitt einer Pressformvorrichtung, wobei der aufnehmende Abschnitt eine Öffnung zum Aufnehmen eines Stempels aufweist, wobei die Gestalt der Öffnung derjenigen des Stempels entspricht;
Anordnen der Biomaterialschicht über der Öffnung des aufnehmenden Abschnitts; und
nachdem das Biomaterial positioniert worden ist, Abwärtsfahren des Stempels, um in der Öffnung aufgenommen zu werden.

## Revendications

1. Capuchon nerveux comprenant :
un corps ayant une extrémité distale, une extrémité proximale comportant une ouverture unique, et une chambre interne entre l'extrémité distale et l'extrémité proximale, le corps comprenant un biomatériau configuré pour se remodeler autour d'une terminaison nerveuse pour former un coussin tissulaire ;
au moins une cloison de séparation à l'intérieur de et fixée à une surface interne de la chambre interne, l'au moins une cloison subdivisant au moins une partie de la chambre interne, l'au moins une cloison de séparation comprenant un biomatériau, et l'au moins une cloison de séparation s'étendant dans une direction longitudinale depuis l'extrémité distale de la chambre interne sur environ 1/4 à environ 3/4 d'une longueur de la chambre interne ;
une cavité de rétention entre l'ouverture unique à l'extrémité proximale et l'au moins une cloison de séparation ; et
au moins une languette s'étendant depuis un côté externe du corps,
une longueur externe du capuchon étant dans une plage de 1 mm à 500 mm.

2. Capuchon nerveux selon la revendication 1, dans lequel l'au moins une cloison de séparation s'étend dans la direction longitudinale sur environ 2/5 à environ 3/5 de la longueur de la chambre interne, ou environ 1/2 de la longueur de la chambre interne.

3. Capuchon nerveux selon la revendication 1 ou la revendication 2, dans lequel l'au moins une cloison de séparation est un séparateur unique.

4. Capuchon nerveux selon la revendication 3, dans lequel le séparateur unique coupe en deux la chambre interne.

5. Capuchon nerveux selon la revendication 1 ou la revendication 2, dans lequel l'au moins une cloison de séparation comprend deux cloisons disposées perpendiculairement l'une à l'autre.

6. Capuchon nerveux selon la revendication 1 ou la revendication 2, dans lequel l'au moins une cloison de séparation comprend deux cloisons ou plus disposées parallèlement les unes aux autres.

7. Capuchon nerveux selon l'une quelconque des revendications 1, 2 ou 6, dans lequel l'au moins une cloison de séparation comprend deux cloisons ou plus non connectées les unes aux autres.

8. Capuchon nerveux selon l'une quelconque des revendications précédentes, dans lequel l'au moins une languette traverse un diamètre de l'extrémité distale du corps.

9. Capuchon nerveux selon l'une quelconque des revendications 1 à 7, dans lequel une largeur de l'au moins une languette est inférieure à un diamètre de l'extrémité distale du corps.

10. Capuchon nerveux selon l'une quelconque des revendications précédentes, dans lequel l'au moins une languette comprend une seule languette.

11. Capuchon nerveux selon l'une quelconque des revendications 1 à 9, dans lequel l'au moins une languette comprend deux languettes ou plus.

12. Capuchon nerveux selon l'une quelconque des revendications précédentes, dans lequel un matériau qui forme l'au moins une languette est le même qu'un matériau qui forme le capuchon.

13. Capuchon nerveux selon l'une quelconque des revendications 1 à 11, dans lequel un matériau qui forme l'au moins une languette est différent d'un matériau qui forme le capuchon.

14. Capuchon nerveux selon l'une quelconque des revendications 1 à 11, dans lequel l'au moins une languette se compose d'un polymère qui est configuré pour se dissoudre et/ou être absorbé par le corps.

15. Capuchon nerveux selon l'une quelconque des revendications précédentes, dans lequel l'au moins une languette s'étend le long d'un axe qui est approximativement parallèle à ou est parallèle à la direction longitudinale de la chambre interne.

16. Capuchon nerveux selon l'une quelconque des revendications 1 à 14, dans lequel l'au moins une languette s'étend le long d'un axe qui est approximativement perpendiculaire à ou est perpendiculaire à la direction longitudinale de la chambre interne.

17. Capuchon nerveux selon l'une quelconque des revendications 1 à 14, dans lequel l'au moins une languette s'étend le long d'un axe qui est à un angle oblique par rapport à la direction longitudinale de la chambre interne.

18. Capuchon nerveux selon la revendication 1, dans lequel le biomatériau est sélectionné dans le groupe constitué d'un polyéthylène de haute densité (HDPE), d'un hydrogel de polyéthylène glycol (PEG), et de protéines purifiées provenant de sources humaines ou animales.

19. Capuchon nerveux selon la revendication 1, dans lequel le biomatériau est sélectionné dans le groupe constitué d'une sous-muqueuse d'intestin grêle (SIG), d'une membrane amniotique, d'un derme, d'un collagène ou d'un fascia décellularisé.

20. Capuchon nerveux selon l'une quelconque des revendications 1 à 8 ou 11 à 19, dans lequel l'au moins une languette s'étend depuis un côté du corps.

21. Capuchon nerveux selon l'une quelconque des revendications 1 à 19, dans lequel l'au moins une languette s'étend depuis l'extrémité distale du corps.

22. Capuchon nerveux selon l'une quelconque des revendications 1 à 7 ou 10 à 19, dans lequel l'au moins une languette se trouve au niveau de ou à proximité de l'ouverture unique dans le corps, et optionnellement, l'au moins une languette s'étend depuis un bord de l'ouverture unique dans le corps.

23. Capuchon nerveux selon l'une quelconque des revendications précédentes, dans lequel un ou les deux d'une longueur du capuchon et d'un diamètre du capuchon sont dans une plage de 1 mm à 100 mm.

24. Procédé de fabrication d'un capuchon nerveux, de préférence selon l'une quelconque des revendications précédentes, comprenant :
le formage sous pression d'une couche de biomatériau, de préférence en forme de tige,
optionnellement dans lequel le formage sous pression de la couche de biomatériau comprend :
le montage d'une couche de biomatériau sur une partie réceptrice d'un appareil de formage sous pression, la partie réceptrice étant pourvue d'une ouverture pour recevoir un poinçon, la forme de l'ouverture correspondant à celle du poinçon ;
la mise en place de la couche de biomatériau pardessus l'ouverture de la partie réceptrice ; et
une fois que le biomatériau a été positionné, l'abaissement du poinçon pour l'introduire dans l'ouverture.
